Europäisches Patentamt

European Patent Office

Office européen des brevets

(11) Publication number: **0 250 137 B1**

(12) EUROPEAN PATENT SPECIFICATION

(45) Date of publication of patent specification: **26.08.92**

(51) Int. Cl.⁵: **G01N 33/558**, G01N 33/543, G01N 33/532, G01N 33/74, //G01N33/94

(21) Application number: **87305027.2**

(22) Date of filing: **08.06.87**

(54) **Colloidal gold immunoassay.**

(30) Priority: **09.06.86 US 872357**

(43) Date of publication of application:
**23.12.87 Bulletin 87/52**

(45) Publication of the grant of the patent:
**26.08.92 Bulletin 92/35**

(84) Designated Contracting States:
**AT BE CH DE FR GB IT LI LU NL SE**

(56) References cited:
**EP-A- 0 007 654
EP-A- 0 093 613
EP-A- 0 158 746
GB-A- 2 111 201
US-A- 4 235 601**

(73) Proprietor: **ORTHO DIAGNOSTIC SYSTEMS INC.
One Johnson & Johnson Plaza
New Brunswick New Jersey 08933-7003(US)**

(72) Inventor: **Mochnal, Dennis M.
18 Hancock Street
Clinton, NJ 08809(US)**
Inventor: **Graham, Henry A.
RD 2 Box 74, Sand Hill Road
Annadale, NJ 08801(US)**
Inventor: **Kang, Jemo
22 Nassau Court
Skillman, NJ 08558(US)**
Inventor: **Chang, Chi-Deu
60 Stella Drive
Bridgewater, NJ 08807(US)**
Inventor: **Chen, Shin
983 Brown Road
Bridgewater, NJ 08807(US)**

(74) Representative: **Jones, Alan John et al
CARPMAELS & RANSFORD 43 Bloomsbury Square
London, WC1A 2RA(GB)**

## Description

Field of the Invention

This invention relates to the field of immunoassays and more particularly provides a new immunoassay using colloidal gold and membranes for detecting molecules, including small molecules such as those containing a single epitopic site, in urine or other body fluids.

Background of the Invention

Many human health conditions can be ascertained by the use of immunologically-based immunoassays. Such assays rely upon the specificity of reaction between immunoglobulins whether monoclonal or polyclonal-based, and their respective binding partners which may be haptens, antigens or other analytes all of which may hereafter be collectively and interchangeably referred to as ligands and ligand binding partners or biologically active molecules generally. Immunoassays are procedures which utilize the specific binding ability between ligands and ligand binding partners in determining the presence or absence of ligands or their binding partners in a fluid sample. Many such procedures are known and include sandwich asays, competitive binding assays and the like. While the instant invention utilizes the basic precepts of such assays, it is not deemed necessary to review in detail such conventionally known procedures.

Horisberger and Rosset, J. Histochem., Cytochem., 25:295-305 [1977], described an agglutination assay for mannan using colloidal gold as a label. While such colloidal gold agglutination assays have recently been commercialized, they have disadvantages associated with their speed, difficulty in determining subjective end points by color and the like.

It is an object of the present invention to employ colloidal gold as a label while avoiding disadvantages associated with Horisberger's agglutination assays.

In U.S. Patent No. 4,313,734, Leuvering describes a new immunoassay procedure involving the use of colloidal metal substances. While those procedures describe the use of a new label, i.e. colloidal metal, all were dependent upon the use of spectrophotometers or atomic flameless spectrophotometers for identifying the presence of the colloidal metal after it had been chemically leached from the site of immunological reaction. As a result, Leuvering's assays were dependent upon cumbersome procedures and expensive instrumentation in order to derive relatively insensitive results. It is not surprising therefore that the leaching procedures described by Leuvering have not found their way into the commercial marketplace which demands fast, sensitive and economical procedures capable of being performed without such dependence upon expensive instrumentation.

It is one aspect of the instant invention to utilize a colloidal gold label such as described by Leuvering but to utilize the label in an entirely new procedure which overcomes all of the deficiencies of the Leuvering procedures.

European Patent Application 0 158,746 assigned to Janssen describes blot overlay assay methods which rely upon diffusion to the surface. While providing a dramatic improvement over Leuvering, the described methods still suffer from excessive time requirements to obtain minimally acceptable sensitivity. Janssen also described new techniques for improving the sensitivity or visualizability of assay results by innovative silver enhancement procedures.

It is an object of the present invention to provide yet additional improvements over the Janssen methods in both sensitivity and speed of assay.

In U.S. Patent No. 3,888,629, Bagshawe described an immunoassay procedure utilizing radioisotopes with a cartridge including a reaction surface in contact with an absorbent material for drawing fluids through the membrane. While the Bagshawe device claimed high sensitivity, it presented a less desirable solution inasmuch as it relied upon isotopic labels. Such labels require sophisticated detection equipment for determining their presence upon the reaction surface. Further, and as is well-known, radioisotopes pose substantial health and procedural difficulties making such procedures generally less desirable.

It is another aspect of the present invention to improve upon the mechanical aspects of the Bagshawe device while avoiding the disadvantages associated with isotopic procedures generally.

U.S. Patent Nos. 4,235,601 and 4,361,537 to Deutsch et al. describe related test devices for determining a particular characteristic of a sample such as for determining the presence of a substance in a fluid sample. The Deutsch et al. device employs a strip element having a plurality of zones for receiving a fluid sample or containing various reagent components. One end of the strip element is then immersed into a developing fluid which flows upward through the capillary passages thereby coming into contact with the sample and conveying the sample in a continuing upward direction through other zones containing various

2

reagents. One such zone might for instance contain a labeled reagent (e.g. zone 14 in FIG. 1) and another zone (e.g. zone 15 in FIG. 1) containing an immobilizing element such as an antibody for retaining the label in that location if the sample applied to the first zone 13 contained the element for which the label and immobilized antibody were specific. Thus, all immunologically reactive components are stored on the strip element, the only liquid reagent being the developing fluid which was described as comprising an aqueous solution of sodium barbitol, hydrochloric acid, thimerosal and disodium ethylenediamine tetraacetate.

It is another aspect of the present invention to utilize an approach similar to Deutsch et al. but to avoid the necessity of using complicated developing solutions as taught by Deutsch et al. while employing superior label systems.

EPA 164,194 describes a similar system using a strip element, however, the point of novelty in that invention appears to be solely directed to the employment of lasers to cut out the strip elements thereby ensuring a uniformly level advancing fluid front.

It is still another aspect of the present invention to provide new immunoassay methods which are substantially insensitive to methods of strip manufacture and thus do not require the methods taught by EPA 164,194.

EPA 143,574 teaches a strip element immunoassay system which employs a binding agent to aggregate RBCs at the air/liquid interface on the bibulous element. The assay relies upon conventional ELISA techniques and goes so far as to state on page 22 that individual labels will not be sufficient to produce the desired sensitivity thereby requiring an enzyme label, each of which can generate a plurality of detectable molecules.

It is still yet another aspect of the present invention to provide immunoassays which utilize similar strip elements but which do not require enzyme labels and which can still be read visually.

It is yet another aspect of the present invention to provide assay procedures which may be interpreted entirely visually without reliance upon instrumentation but which may also be used with instrumentation if higher sensitivity is desired or in alternative quantitation modes.

It is yet a further aspect of the present invention to provide assays which may be produced economically, performed expeditiously, and derive the required sensitivity necessary to perform satisfactorily in all medical environments in both the stat and batch modes.

## Summary of the Invention

In keeping with the aspects and goals of the present invention, there is provided a new immunoassay procedure for determining the presence of antibodies or ligands, such as drugs of abuse in fluid samples such as urine, cerebrospinal fluid, saliva, whole blood, blood components or other aqueous body discharges or solubilized discharges. The assay is performed with a matrix surface having a tortuous path therethrough and having two ends and having either a ligand or the ligand binding partner immobilised within the membrane, depending on whether a competitive or sandwich type assay is to be performed. The matrix surface or membrane is preferably bibulous in nature thereby providing capillary passages for the flow of fluids therethrough thereby bringing the fluid into contact with the ligand or ligand binding partner immobilised within the membrane at one or more locations. A liquid reagent is thereafter provided which contains a ligand binding partner or ligand, said ligand binding partner or ligand being labelled directly or indirectly with colloidal gold, one end of said membrane strip is contacted with said sample and, simultaneously or successively, with said liquid reagent whereby an immunological reaction is carried out as the sample and the reagent move from said one end to the other end of the membrane strip. The membrane is observed for the presence of colour, the distance of which from said one end being correlatable to the presence of colloidal gold and of said ligand in said sample.

Most preferably, the ligand containing sample and a colloidal gold labelled biologically active molecule are pre-mixed and then brought into contact with one end of the bibulous strip. The mixture is then drawn through the matrix by capillary action, allowing the localisation of colloidal gold in areas having an immobilised binding partner while washing away unbound colloidal gold in the process. With immunoglobulin tests, it is most preferred sequentially to add sample and colloidal gold labelled reagent with intermediate wash steps. Surprisingly, colloidal gold remaining in the matrix results in the presence of a very strong, visually detectable coloured spot.

This invention also provides a kit for use in an assay for the detection of a ligand in a fluid sample which comprises a membrane having immobilised therethrough continuously or in separate zones a ligand binding partner or a ligand for binding respectively to the ligand or ligand binding partner and a container having a colloidal gold labelled ligand or ligand binding partner for binding to the ligand binding partner on the membrane in a competitive assay or to the ligand in the fluid sample in a sandwich assay.

As a result of the instant invention, fever liquid reagents are required than in conventional assays, e.g. one reagent with an optional wash fluid as opposed to four or more reagents required with ELISA procedures. No instruments are required in order to read the results but may be used optionally to measure the presence of colloidal gold based on its ability to scatter or absorb light. And, the entire procedure including all handling steps and a single incubation step can be performed generally in less time than comparable assays, while still readily achieving great sensitivity. In the case of LH, a sensitivity of 20 mIU/ml urine or better is routinely obtained with a two-three drop sample and a total test performance time of approximately five minutes, inclusive of incubation and handling times. Another advantage provided is the elimination of incubation related errors since there are no requisite wash steps with assays for antigens and haptens. Further, completion of the test results in a stable endpoint.

Detailed Description of the Invention and Best Mode

The inventors hereof have surprisingly discovered that the unobvious combination of a strip matrix or membrane surface and immunoassays employing colloidal gold as a label, can result in extremely rapid and sensitive assays providing results which may be visually detected. Such an assay is especially useful for large and small molecules, and for antibodies but is not so limited. Such assays have not heretofore been possible. This is particularly true since conventional surface assays just don't provide enough colloidal gold to be visually detectable and prior methods have required ELISA techniques or instruments to detect fluorescent labels. Surprisingly, a membrane surface provides many "layers" which, rather unexpectantly, provide sufficient concentration of colloidal gold to be visually detectable. This observation has figured prominantly in a colloidal gold membrane assay (COGMA) described in European patent application No. 87305026.4 (Publication N° 0258963) claiming priority from USSN 872 355 - ORD 63) filed contemporaneously herewith. Unlike that assay, the instant assay generally achieves higher sensitivity per ml of sample because all of the sample flows past areas of immunological component in the test strip rather than just through certain sections as in COGMA. As a related advantage, less colloidal gold labeled component is required in order to achieve comparable sensitivity.

The instant assay is most efficaciously performed utilizing a tortuous, e.g. bibulous, membrane strip which permits the capillary flow of fluids therethrough and to which immunologically active components may be attached. By using such a tortuous membrane, reaction kinetics normally associated with solution phase reactions become possible despite the fact that one of the immunological reactions takes place at a solid phase surface. Further, such materials allow for flexibility with respect to orientation since the direction of flow is not critical. While a number of types of filter membranes may be employed including for example activated Whatman™ 31 ET Chromatography Paper, activated nylon 66 (e.g. Biodyne™ from Pall), and activated PVDF (polyvinylidine fluoride such as Immobilon™ from Millipore), the most preferred material is the Whatman material for ease of handling.

Using LH (leutinizing hormone) as an example of the utilization of the present invention in a sandwich assay format, one would attach LH specific antibody to the membrane. While the membrane is preferably a strip having dimensions such as 5 x 90 mm, the instant invention permits great freedom regarding physical construction. The anti-LH antibody may be attached either continuously throughout the membrane strip or in equally spaced zones along the membrane strip.

The sample, such as a urine, containing the suspected LH is mixed with a liquid reagent comprising a colloidal gold labeled (directly or indirectly through an intermediate antibody or other linkage) anti-LH antibody which is specific for a different epitopic site than is the membrane immobilized antibody.

The mixture of sample and reagent is then ideally applied to one end of the exposed membrane and allowed to migrate through the strip. While the instant assay permits extraordinary variation in the volume, it has been noted that increased volumes, limited only by the absorbent capacity of the membrane and the amount of reagent, may be advantageously used to improve the assay's sensitivity.

Continuing with the LH example, LH present in female urine (generally associated with the onset of ovulation) is captured by labeled antibody during the mixture step and then becomes attached within the membrane to the anti-LH antibodies immobilized therein. The derivation of such antibodies, either of polyclonal or monoclonal nature, is an art well-known and need not be described in detail here. From a specificity and manufacturing viewpoint, the most preferred antibodies will be monoclonal antibodies derived through the methods of Kohler and Millstein first described in 1975 and published extensively elsewhere.

Unattached colloidal gold labeled reagent as well as the remaining portions of the sample including the fluid of the sample and non-LH ligands are drawn throughout the remaining portion of the membrane. As a result one will be able to observe the colloidal gold (a bluish-red color) for a distance from the end to which sample was applied (e.g. the end immersed in the sample-reagent mixture). If the immobilized antibody was applied continuously throughout the strip, then some portion of the strip will 30 show continuous color. The length of the membrane showing color will be proportional to the amount of LH present in the sample. Similarly, different zones will show "steps" of color and the number of steps or zones of color can be correlated to LH concentration. Such zones are preferred in that they act as concentrations of the labeled component thereby allowing lower concentrations of labeled components to be used while retaining, or even augmenting the contrast between the zones and the background areas on the strip element. As a related advantage, wash steps are obviated since the labeled component is diluted by the sample, all of which flows by the reaction zone. More LH in the sample will result in a larger distance or a greater number of colored zones as well as greater intensity of color in the colored zones.

Since in concentrations greater than normal background levels (e.g. equal to or less than 10 mIU/ml urine) LH elevated should be monitored over a number of days (e.g. 6-9) during the mid-menstrual cycle, a preferred commercial product or kit will include an equal number (6-9) of membranes plus one or more reagent bottles containing the colloidal gold labeled reagent and ideally also instructions. The kit may be expanded to nine tests for those women suffering irregular menstrual cycles. Optionally, an extra positive control (LH either labeled directly with colloidal gold or for mixture with colloidal gold labeled antibody reagent) with test strip may also be provided. Alternatively, an internal control could be provided by having an additional zone or bard of LH on the membrane strip. Optionally, the LH (or equivalent ligand) can be calibrated to known concentrations in order to provide a standard color comparative zone. Such internal comparative zones are additional novel aspects of the present invention. While one may now utilize an optional wash step to remove unbound material, the most preferred embodiment based on procedural simplicity advantageously saves time and dispenses with such wash step as being unnecessary in order to obtain the clinically relevant sensitivities.

Experimentation has shown a substantial variety of colloidal gold sizes as measured by the optical density ratio of 540 nm over 600 nm may be utilized including from about 1.7 (large particles) to 3.0 (small particles) with a preferred ratio within the range of 2 to 2.5. While 1.7 is associated with the size of the largest particle tested, there is no reason to believe larger particles, albeit more difficult to make, could not be used satisfactorily. Colloidal gold particles in the preferred size or optical ratio range may be visually observed as a reddish blue color when in solution. Preferred methods of colloidal gold formation and their attachment to immunoglobulins are described later.

While the best mode contemplates direct labeling of the ligand or ligand binding partner with colloidal gold, it is also contemplated that equivalent indirect labeling may be employed and even preferred in certain circumstances. Such indirect labeling would typically utilize a third immunoglobulin which is itself labeled with the colloidal gold and is specifically reactive with the second immunoglobulin, in turn specific for the ligand to be detected. Such methods are particularly useful for antibody tests using anti-globulin labeled colloidal gold. Again, indirect labeling procedures are subjects readily understood by skilled practitioners in the art.

While it is most preferred to allow the membrane strip to develop for a period of time, e.g. five minutes, following contact with the ligand containing sample - colloidal gold labeled reagent mixture, no special incubation conditions are required, room temperature being perfectly adequate. Results may then be directly observed, at any convenient time thereafter. It will be readily understood that immunoglobulin tests will preferably have sequential additional of the sample, intermediate wash and colloidal gold labeled reagent to the strip membrane.

It will be noted that as a result of the instant invention, fewer liquid reagents are required in order to perform an assay (indeed, most assays will require only one reagent having a biologically active component), and fewer steps need be performed. As a result, the typical reaction may often be run in less than five minutes for ligands or ten minutes for antibodies while still obtaining levels of sensitivity comparable to the best conventional assays but, unlike those other assays, providing visually detectable, unequivocal results with astounding speed.

Other advantages are provided by the instant invention. Neither the reagents, nor the membrane containing a biologically active molecule need be at room temperature when the assay is performed. This is in stark contrast with conventional ELISA procedures which are highly temperature sensitive.

The invention further avoids the disadvantages associated with ELISA techniques including the characteristic criticality associated with timing of steps, pH, and temperature of solutions. In contrast, the instant invention avoids such limitations.

Perhaps the most surprising aspect of the instant invention is the fact that a positive reaction may be visually detected at all. While this aspect of the invention is as yet still not fully understood by the inventors hereof, it is believed that the multi-layered nature of the membrane coupled with the light scattering properties of the colloidal gold results in accumulation, if not amplification of the visual effect of the presence of colloidal gold.

Other non-antibody molecules can be tested in a fashion similar to LH. For example, one may detect small molecules having a single epitopic site by competitive assay procedures. Such small molecules include drugs of abuse, digoxin, theophylline and many others whose presence has clinical relevance. In the case of theophylline (THEO) in a blood sample, the test would be performed as follows: specific anti-THEO antibodies are attached covalently on the membrane strip in narrow bands equally spaced along the length of the strip. One end of the membrane strip, thus capable of binding theophylline immunologically, is inserted into a tube containing a premixed suspension of blood sampe and colloidal gold labeled THEO. The unlabeled THEO from the sample will thus compete with the colloidal gold labeled THEO for antibody binding sites on the paper. As a result, the height of the colored visual signal is directly proportional to the theophylline concentration in the sample.

Alternatively, one may attach theophylline directly onto the membrane strip in narrow bands (crosswise) along the entire length of the strip. Alternatively, in both this and the foregoing example, the biologically active molecule may be attached continuously along the length of the membrane strip rather than in zones. One end of the membrane strip, capable of binding anti-theophylline antibody immunologically, is inserted into a tube containing a premixed suspension of the blood sample containing unlabeled theophylline, and colloidal gold labeled anti-theophylline antibody. Again, the height of the visual signal is directly proportional to the theophylline concentration in the sample.

In a number of instances, the presence of antibodies is used to determine exposure to disease causative organisms. Such tests include for instance Rubella IgG, Rubella IgM, Toxoplasmosis IgG and IgM, Cytomeglovirus IgG and IgM, HTLV III, Anti-EBNA, Mononucleosis, Anti-Core, HAA, Herpes, and Anti-Streptolysin O. Generally, antibody tests will be accomplished in substantially the same manner as the small molecule test in that the ligand or antigen itself will be immobilized on the membrane for reaction with the antibody in the patient sample. The colloidal gold labeled reagent may then be suitably selected as an anti-human IgG or IgM as appropriate. The height of the color will then be proportional to the amount of antibody present in the sample.

The instant invention may also be employed to detect the presence of infectious disease agents including microorganisms such as Streptococcus, Chlamydia, Gonococcus or viral organisms such as hepatitis and the like.

Most preferred embodiments of the instant invention will have the capacity to perform multiple tests using the same sample. In such embodiments, a plurality of zones will be used, each with different specificity for sample ligands or antibodies. Contemplated is the simultaneous performance of a test for antibodies present in blood samples, specific for hepatitis core, HTLV-III (AIDS), CMV and the like. The TORCH series (toxoplasmosis, rubella, cytomeglovirus and herpes) is a prime example. In such instances, the control zone(s), if used, should preferably be placed at the top (e.g. furthest from sample addition) in order to ensure that all prior zones have worked properly and have not been neutralized in the absence of gold. These and other aspects of the present invention will become clear upon study of the accompanying examples.

Example 1 - Preparation of Colloidal Gold Sol

All glassware surfaces which came into contact with colloidal gold sold were siliconized utilizing 1% Silwet 720 (Union Carbide) by soaking 10 minutes and then rinsing with Distilled Water (D.W.). ('Silwet 720' is a registered Trade Mark) 1 liter filtered distilled water was heated to 100°C. for at least 10 minutes. 10 ml of 1% gold chloride (Aldrich) in Milli-Q filtered D.W. was added to the reactor and mixed for one minute. ('Milli-Q' is a registered Trade Mark) 10 ml of 34 mM sodium citrate or 10 ml of 64 mM sodium malate or malic acid at pH 4.2 was added to the reactor to act as a reducing agent and rapid mixing continued for 20 minutes. A color change indicated successful formation of a gold sol. The heat source was removed and the reactor cooled to 15-30°C. 1 ml of 1% PEG 20,000 was added and pH of the sol and the reactor was adjusted to 7.1 ± .1 pH with the addition of 0.1 molar $K_2CO_3$. The colloidal gold O.D. may be measured at 540 and at 600 nm. In this form, the gold sol is suitable for coupling to antibody. Colloidal gold thusly produced has a size of about 40 nm to about 50 nm as determined by the OD 540/600 ratio, such size being dependent on the chemical reducing conditions.

Example 2 - Colloidal Gold--Antibody Coupling

Procedure 1 - Adsorptive Coupling

Antibody was dialyzed into 0.01 M HEPES (N-2-hydroxyethyl piperazine N'-2-ethane sulphonic acid) buffer solution at pH 7.1 using 12,000-14,000 molecular weight cut off dialysis tubing and 300 ml solution/ml of antibody for 18 hours and then filtered through a 0.45 $\mu$m SWINEX™ filter. Protein concentration of antibody may be obtained by measuring the antibody's absorption at 280 nm and diluted to a concentration of 3-4 mg/ml with 0.01 HEPES pH 7.1 to obtain optimal antibody--colloidal adsorption coupling as determined by the antibody to be labeled. With the sol still present in the reactor from Example 1, the thusly filtered and diluted antibody was added while the sol was stirred at room temperature. Stirring continued for approximately 30 minutes whereupon (0.1 x sol volume) mls of buffer (D) comprising 0.01 M HEPES; 0.3 M D-MANITOL; 0.05% PEG 20,000; 0.1% BSA--RIA grade 0.05% sodium azide was added. Stirring continued at room temperature for 30 minutes. The solution was then transferred to a high speed centrifuge, centrifuged and the supernatent removed. The sol was then washed four times utilizing the aforedescribed buffer D and resuspended to 1/10 the starting sol concentration, filtered through a 0.45 $\mu$m membrane and stored in polypropylene at 2-8°C.

Preferred Procedure 2 - Covalent Coupling

Mix equivalent weight amount of BSA (100 mg BSA per 100 mg SOL) into SOL generated from Example 1 with pH preadjusted to 6.0. Stir for 2 hours at room temperature. Filter through 0.22 micron membrane filter to remove large particles. Measure OD 540 and 600 nm and calculate OD ratio; OD 540/OD 600 of acceptable material should be 2.50 ± 0.30. Take 200 ml of thusly prepared BSA sol, centrifuge (25,000 G for 30 minutes) and wash once with distilled water. Mix the washed particles with 1% wt/vol. glutaraldehyde and stir for 2 hours. Centrifuge (25,000 G for 30 minutes) and wash three times with distilled water. Mix the activated and washed particles with 2 mg of purified appropriate monoclonal IgG in pH 7.4 phosphate buffer and stir overnight at 2-8°C. Add 2.5 mg sodium borohydride to stabilize the coupling. Stir 30 minutes, and quench with 5 ml pH 8.0 glycine BSA buffer. Centrifuge (25,000 G for 30 minutes) and wash three times with phosphate buffered saline (pH 8.0). Resuspend and adjust dye concentration with final pH 8 buffer (50 mM triethanolamine, 100 mM NaC1, 0.2% BSA, 0.1% $NaN_3$) to about 10 O.D. at 540 nm. Filter through 0.22 micron filter and store at 2-8°C.

Example 3 - Membrane Preparration

Whatman 31ET paper (90 x 152 mm) was immersed in pyridine solution (Baker) containing 0.2 M 1,1'-carbonyldiimidazole and incubated at room temperature for 60 minutes. The membrane strip was then washed with tetrahydrofuran (Baker). The membrane was placed on a glass plate (TLC plate) and secured with tape for ease of handling. Using a LINOMAT™ applicator, 0.8 $\mu$l/cm (2 $\mu$l/inch) of mouse anti-theophylline monoclonal antibody was applied horizontally at evenly spaced locations along the length of the strip. The membrane was removed from the plate and incubated at room temperature for 30 minutes. Blocking solution comprising 0.5% w/v casein Type 1, 0.1% w/v TWEEN™ and PBSS was applied over the membrane and incubated for 90 minutes at 37°C. The membrane was washed with wash buffer comprising 0.1% w/v TWEEN in PBSS at pH 7.3 for 15 minutes at room temperature. The membrane strip was immersed in 0.5% w/v polyvinyl alcohol solution (DuPont) and incubated for 30 minutes. Thereafter the membrane was dried at 37°C. and stored in a moisture excluding container such as an aluminum or plastic bag containing desiccant at 2-30°C. until use. For antigen coated membrane (in theophylline examples) theophylline-BSA conjugate was applied in substitution for the antibody.

Example 4 - Sensitivity For LH In Urine

20 $\mu$ls of colloidal gold labeled anti-LH antibody was added to two drops (100 $\mu$ls) of urine sample and mixed in a test tube. A membrane strip prepared pursuant to Example 3 having anti-LH antibody applied thereto had one end dipped into the test tube. The following results using an LH standard prepared in concentration ranges from 0 mIU per ml to 200 mIU per ml was tested and the following results observed:

## Sensitivity mIU/ml LH

| Standards | 0 | 5 | 15 | 40 | 100 | 200 |
|---|---|---|---|---|---|---|
| Observed Reaction | − | − | − | + | + | + |

Example 5 - Patient Samples

The procedure of Example 4 was followed utilizing urine samples from two women having 28 day menstrual cycles. The samples were collected over a five day period and the test results were verified by Advanced Care's Ovutime Test™ (Ortho Pharmaceutical Corporation) and the following results observed:

### Patient A

| Day in cycle | 9 | 10 | 11 | 12 | 13 |
|---|---|---|---|---|---|
| Test Result | − | − | + | + | − |
| Ovutime Result | − | − | + | + | − |

### Patient B

| Day in cycle | 9 | 10 | 11 | 12 | 13 |
|---|---|---|---|---|---|
| Test Result | − | − | + | + | − |
| Ovutime Result | − | − | + | + | − |

Example 6 - Ovulation Test With Built-in Test Control

A membrane strip was prepared pursuant to Example 4 except that the strip had two reagent bands. The lower band was coated with anti-LH antibody (forming the test band) and the upper band coated with LH (or hCG which reacts similarly due to cross-reactivity) serving as a control band. The colloidal gold labeled anti-LH antibody was prepared pursuant to Example 2. The procedure was as follows: 20 $\mu$ls of colloidal gold labeled anti-LH antibody was added to a test tube along with 100 $\mu$ls of urine sample and mixed. One end of the aforedescribed membrane strip was inserted into the mixture in the test tube and allowed to develop for five minutes. The results observed were as follows:
The same two women's urine sample A and B tested in Example 1 were tested conforming the same result. In addition, this test shows procedural test control band for all tests if it is done properly.

## Patient A

| Day in cycle | 9 | 10 | 11 | 12 | 13 |
|---|---|---|---|---|---|
| Test band | − | − | + | + | − |
| Control band | + | + | + | + | + |
| Reference (Ovutime) | − | − | + | + | − |

## Patient B

| Day in cycle | 9 | 10 | 11 | 12 | 13 |
|---|---|---|---|---|---|
| Test Band | − | − | + | + | − |
| Control Band | + | + | + | + | + |
| Reference (Ovutime) | − | − | + | + | − |

The foregoing procedure was repeated with the colloidal gold labeled antibody lyophilized in the test tube. Three drops (100 μls) of urine was added to the test tube and mixed and thereafter one end of the membrane strip was inserted and developed for five minutes. The observed results were identical.

The test was repeated with clinical samples:

|  | Number of Samples | Number Positive | Number Negative |
|---|---|---|---|
| Urines From Women | 24 | 6 | 18 |

Ovutime™ Reference Test was performed on the same samples and the results confirmed.

Example 7 - Inhibition Assay

BIODYNE membrane (pore size 3.0 μm) were placed in a monoclonal anti-hCG solution (1.2 ug/ml) buffered with 0.02M phosphate buffered saline solution (PBSS). The membrane was incubated at room temperature for 30 minutes under constant agitation. The membrane was removed from the antibody solution and washed by filtration with a PBSS-0.1% Tween 20 wash solution. The washed membranes were placed in a blocking solution of 0.5% dry milk in PBSS and incubated at 37°C for one hour. The membranes were washed by filtration with PBSS-0.1% Tween wash solution, dried at 37°C and stored in a moisture excluding container containing desiccant at 2-30°C. Monoclonal anti-hCG antibody was labeled with colloidal gold. These antibodies were then saturated with hCG producing a label-antibody-analyte complex. The previously prepared membranes were cut into strips 50 mm x 6 mm. 500 ul urine sample was mixed with 100 ul of marker. The strips were placed into sample solution (to a depth of 5 mm) and the fluid allowed to migrate to top of strip. The distance the colloidal dye migrated from the surface of the sample solution was visually observed. The results observed were as follows:

| Sample HCG (mIU/ml) | 0 | $3 \times 10^2$ | $3 \times 10^3$ | $3 \times 10^4$ | $3 \times 10^5$ | $3 \times 10^6$ | $3 \times 10^7$ |
|---|---|---|---|---|---|---|---|
| Distance Migrated (mm) | 0 | 0 | 0 | 7 | 15 | 35 | 45 (Top) |

Example 8 - Procedure Using an Alternative Label to Colloidal Gold

The procedures of Example 7 were repeated except that FITC was used to label the monoclonal anti-hCG antibody and results were observed by illuminating the strips with FITC, (fluorescein thiocyanate) The following results were obtained:

| Sample HCG (mIU/ml) | 0 | $3 \times 10^2$ | $3 \times 10^3$ | $3 \times 10^4$ | $3 \times 10^5$ | $3 \times 10^6$ | $3 \times 10^7$ |
|---|---|---|---|---|---|---|---|
| Distance Migrated (mm) | 0 | 0 | 3 | 12 | 17 | 32 | 45 (Top) |

Example 9 - Theophylline Test

A membrane of 90 x 5 mm was prepared substantially pursuant to the procedures set forth in Example 3 except that anti-theophylline antibody was coated in 11 bands. Colloidal gold-BSA-theophylline was prepared pursuant to Example 2 (e.g. theophylline, chemically coupled with amide bonds to BSA, was substituted for the monoclonal antibody) and the following procedures performed: 20 $\mu$ls of the colloidal gold labeled reagent was added to a test tube along with 130 $\mu$ls of serum sample and mixed. One end of the membrane strip was inserted into the mixture and developed for 10 minutes and then read. Theophylline standards were prepared in concentration ranges from 0 $\mu$gs/ml to 40 $\mu$gs/ml and a control chart (standard concentration versus band height) was prepared from the standard run. The following results were observed.

## Control Chart

| Concentration | 0 | 2.5 | 5.0 | 10.0 | 20.0 | 40.0 |
|---|---|---|---|---|---|---|
| Height of band (mm) | 0 | 7 | 12 | 22 | 37 | 53 |

|  | Height (mm) | Value ($\mu$g/ml) |
|---|---|---|
| Control (1) | 13 | 5 |
| (2) | 6 | 2.5 |

It will be noted that the presence of multiple bands assists in providing a clear contrast between the signal and the background thereby enhancing contrast and facilitating interpretation of results. The test results were not time dependent and remained observable at any time following development. Further, the ability to prepare control charts allows the quantitation of theophylline concentration in those instances when qualitative results (e.g. above a predetermined height) is insufficient.

Example 10 - Urine Pregnancy Test With Built-In Control

A membrane strip was prepared pursuant to Example 4 except that the strip had two reagent bands. The lower band was coated with anti-hCG antibody (forming the test band) and the upper band coated with hCG serving as a control/calibration band. The colloidal gold labeled anti-hCG antibody was prepared pursuant to Example 2. The procedure was as follows:

40 $\mu$ls of colloidal gold labeled anti-hCG antibody was added to a test tube along with 100 $\mu$ls of urine sample and mixed. One end of the aforedescribed membrane strip was inserted into the mixture in the test tube and allowed to develop for five minutes. The results observed were as follows:

|  | Number of Samples | Number Positive (Two bands) | Number Negative (One band) |
|---|---|---|---|
| Urines From Pregnant Women | 8 | 8 | 0 |
| Urines from Non-Pregnant Women | 3 | 0 | 3 |

The test results were in complete agreement (100%) with Hybritech's TANDEM ICON™ hCG Test.

Example 11 - Whole Blood Pregnancy Test With Built-In Control

A membrane strip (90 x 10 mm) was prepared pursuant to Example 10 and tested accordingly with whole blood samples. The procedure was as follows:

10 $\mu$ls of whole blood sample was spotted on the membrane at the position between the test band and the bottom end. The membrane strip was then inserted into a test tube containing a mixture of 40 $\mu$ls of colloidal gold labeled anti-hCG antibody and 160 $\mu$ls of buffer (0.1 M PBS plus 5% BSA, 0.05% Tween-20). After ten minutes, the results were observed as follows:

|  | Number of Samples | Number Positive (Two bands) | Number Negative (One band) |
|---|---|---|---|
| Whole Blood From Pregnant Women | 3 | 3 | 0 |
| Whole Blood from Non-Pregnant Women | 2 | 0 | 2 |

The test results were in complete agreement (100%) with Hybritech's TANDEM ICON™ hCG Test.

Example 12 - Rubella Antibody Test With Built-In Control

11

A membrane strip was prepared pursuant to Example 6 except that the strip had two reagent bands. The lower band was coated with rubella antigen (Immuno Search, Toms River, NJ), and the upper band coated with human IgG serving as a control/calibration band. The colloidal gold labeled mouse anti-human antibody was prepared pursuant to Example 2. The procedure was as follows:

10 $\mu$ls of serum sample was diluted with 25 $\mu$ls of Buffer A (0.1M PBS plus 5% BSA, 0.05% Tween-20) in a test tube. One end of the aforedescribed membrane strip was inserted into the test tube and allowed to develop for one minute. Another 25 $\mu$ls of Buffer A was added to the test tube and the strip was further developed for two minutes. Finally, the strip was transferred to a second test tube containing a mixture of 30 $\mu$ls of colloidal gold labeled mouse anti-human antibody and 100 $\mu$ls of Buffer A. The strip was allowed to develop for seven minutes and the results observed were as follows:

| Number of Samples | Number Positive (Two bands) | Number Negative (One band) |
| --- | --- | --- |
| 7 | 5 | 2 |

The test results were in complete agreement (100%) with an ELISA Rubella test.

Another aspect of the present invention concerns the flexibility to build into the membrane strip a feature for ensuring that the liquid reagents and the biologically active molecules on the membrane strip are active, and that the assay steps have been performed properly including the addition of aqueous sample. This is particularly useful for immunoglobulin tests and is more specifically described in copending European patent application No. 87305028.0 (Publication N° 0249418) (claiming priority from USSN 872 358 ORD 66) filed contemporaneously herewith. In summary, the membrane strip would incorporate a reaction zone having an immobilized component capable of specifically reacting with a sample component which is ubiquitous in all similar samples from different sources. Thereafter, the labeled component is ideally chosen so that it can react both with the immunoglobulin or other ligand to be determined and the ubiquitous sample component. It will thus be seen that an internalized control is performed as an integral part of the assay.

From the foregoing, it will be readily perceived by one skilled in the art that numerous alterations may be made with regard to the selection of immunologically active components, the membranes, and the liquid reagent while still employing the essence of the invention comprising the combination of a colloidal gold labeled reagent in an immunoassay performed in a matrix membrane by flowing materials through the membrane to obtain a visually detectable result.

## Claims

1. A method for detecting a ligand in a sample comprising the steps of:
   a) providing a membrane strip having a tortuous path therethrough, said membrane having two ends and having a ligand or a ligand binding partner immobilised within the membrane;
   b) providing a liquid reagent containing a ligand binding partner or ligand, said ligand binding partner or ligand being labelled directly or indirectly with colloidal gold;
   c) contacting one end of said membrane strip with said sample and, simultaneously or successively, with said liquid reagent whereby an immunological reaction is carried out as the sample and the reagent move from said one end to the other end of the membrane strip; and
   d) observing said membrane for the presence of colour, the distance of which from said one end being correlatable to the presence of colloidal gold and of said ligand in said sample.

2. The method of claim 1, wherein:
   in step b) a ligand, labelled directly or indirectly with colloidal gold, which competes with the ligand in the sample for the ligand binding partner is used; and
   the reagent and the sample are mixed prior to their simultaneous application to the one end of the strip membrane.

3. The method of claim 1, wherein the ligand is an antigen having a single epitopic site; the ligand in step a) is an antigen having the same immunological reactivity as said sample antigen; the ligand binding partner in step b) is an antibody, labelled directly or indirectly with colloidal gold, which is specific for said sample antigen; and the reagent and the sample are mixed prior to their simultaneous application to the one end of the strip membrane.

4. The method of claim 1, wherein:
the ligand in the sample is an antibody;
the ligand binding partner in step a) is an antigen for which the antibody is specific; and
the ligand binding partner in the reagent is an immunoglobulin, labelled directly or indirectly with colloidal gold, which is specific for said antibody.

5. The method of claim 1, wherein:
the ligand in the sample is an antigen having at least two epitopic sites;
the ligand binding partner in step a) is an antibody which is specific for a first epitopic site on the antigen; and
the ligand binding partner in step b) is an antibody, labelled directly or indirectly with colloidal gold, which is specific for a second epitopic site on the antigen.

6. The method of claim 1, wherein:
the ligand in the sample is an antigen having at least two epitopic sites;
the ligand in step a) is an antigen having the same immunological reactivity as said sample antigen;
the ligand binding partner in step b) is an antibody, labelled directly or indirectly with colloidal gold, which is specific for the antigens; and
the reagent and the sample are mixed prior to their simultaneous application to the one end of the strip membrane.

7. The method of any one of claims 1 to 3, 5 and 6 wherein the sample antigen is lutinising hormone (LH).

8. The method of any one of claims 1 to 7, wherein said colloidal gold has an optical density ratio of 1.7 to 3.0 measured as the ratio of the optical density at 540 nm to the optical density at 600 nm.

9. The method of claim 8, wherein the ratio is 2 to 2.5.

10. The method of any one of claims 1 to 9, wherein the membrane strip comprises activated Nylon 66, activated bibulous paper, or activated polyvinylidine difluoride.

11. The method of any one of claims 1 to 10, wherein the one end of the membrane strip is conctacted with a wash solution after it has been contacted with the sample.

12. The method of any one of claims 1 to 11, further comprising the step of incubating said membrane at room temperature for a period of time less than about 10 minutes prior to said observing step.

13. The method of any one of claims 1 to 12, wherein the entire method is performed in less than about 30 seconds.

14. The method of any one of claims 1 to 13, wherein said membrane strip further comprises a calibrator for said ligand to be detected.

15. The method of any one of claims 1 to 14, wherein said membrane strip has a plurality of different ligand binding partners immobilised within the membrane in separate zones whereby a plurality of ligands may be detected.

16. A kit for use in an assay for the detection of a ligand in a fluid sample according to claim 1 comprising:
a membrane having immobilised therethrough continuously or in separate zones a ligand binding partner or a ligand for binding respectively to the ligand or ligand binding partner; and a container

EP 0 250 137 B1

having a colloidal gold labelled ligand or ligand binding partner for binding to the ligand binding partner on the membrane in a competitive assay or to the ligand in the fluid sample in a sandwich assay.

**Patentansprüche**

1. Verfahren zum Nachweisen eines Liganden in einer Probe, welches die folgenden Stufen umfaßt:
   a) Zur-Verfügung-Stellen eines Membranstreifens, der von einem gewundenen Pfad durchsetzt wird, wobei diese Membran zwei Enden hat und einen Liganden oder einen Liganden-Bindungspartner aufweist, der innerhalb der Membran immobilisiert ist;
   b) Zur-Verfügung-Stellen eines flüssigen Reagens, welches einen Liganden-Bindungspartner oder einen Liganden enthält, wobei dieser Liganden-Bindungspartner oder Ligand direkt oder indirekt mit kolloidalem Gold markiert ist;
   c) Inberührungbringen von einem Ende des Membranstreifens mit der Probe und - gleichzeitig oder darauffolgend - mit dem flüssigen Reagens, wodurch eine immunologische Reaktion in dem Maße durchgeführt wird, als die Probe und das Reagens von dem einen Ende zu dem anderen Ende des Membranstreifens wandern; und
   d) Beobachten der Membran auf das Vorliegen von Farbe, deren Abstand von dem einen Ende mit dem Vorliegen von kolloidalem Gold und des Liganden in der Probe korrelierbar ist.

2. Verfahren nach Anspruch 1, wobei in der Stufe b) ein direkt oder indirekt mit kolloidalem Gold markierter Ligand verwendet wird, welcher mit dem Liganden in der Probe un den Liganden-Bindungspartner in Wettbewerb tritt; und wobei das Reagens und die Probe vor ihrem gleichzeitigen Aufbringen auf das eine Ende des Streifens miteinander vermischt werden.

3. Verfahren nach Anspruch 1, wobei der Ligand ein Antigen ist, das eine einzige epitope Stelle aufweist; der Ligand in der Stufe a) ein Antigen ist, das die gleiche immunologische Reaktivität wie das Proben-Antigen aufweist; der Liganden-Bindungspartner in der Stufe b) ein Antikörper ist, der direkt oder indirekt mit kolloidalem Gold markiert ist, und der spezifisch für das Proben-Antigen ist; und wobei das Reagens und die Probe vor ihrem gleichzeitigen Aufbringen auf das eine Ende der Streifenmembran miteinander vermischt werden.

4. Verfahren nach Anspruch 1, wobei:
   der Ligand in der Probe ein Antikörper ist;
   der Liganden-Bindungspartner in der Stufe a) ein Antigen ist, für welches der Antikörper spezifisch ist; und
   der Liganden-Bindungspartner in dem Reagens ein Immunglobulin ist, das direkt oder indirekt mit kolloidalem Gold markiert ist, und welches für diesen Antikörper spezifisch ist.

5. Verfahren nach Anspruch 1, wobei
   der Ligand in der Probe ein Antigen ist, das wenigstens zwei epitope Stellen aufweist;
   der Liganden-Bindungspartner in der Stufe a) ein Antikörper ist, der spezifisch für eine erste epitope Stelle auf dem Antigen ist; und
   der Liganden Bindungspartner in der Stufe b) ein Antikörper ist, der direkt oder indirekt mit kolloidalem Gold markiert ist, und der spezifisch für eine zweite epitope Stelle auf dem Antigen ist.

6. Verfahren nach Anspruch 1, wobei
   der Ligand in der Probe ein Antigen ist, welches wenigstens zwei epitope Stellen aufweist;
   der Ligand in der Stufe a) ein Antigen ist, das die gleiche immunologische Reaktivität wie das Proben-Antigen aufweist;
   der Liganden-Bindungspartner in der Stufe b) ein Antikörper ist, der direkt oder indirekt mit kolloidalem Gold markiert ist, und der spezifisch für die Antigene ist;
   und wobei das Reagens und die Probe vor ihrem gleichzeitigen Aufbringen auf das eine Ende der Streifenmembran miteinander vermischt werden.

7. Verfahren nach einem der Ansprüche 1 bis 3, 5 und 6, wobei das Proben-Antigen Luteinisierendes Hormon (LH) ist.

14

**8.** Verfahren nach einem der Ansprüche 1 bis 7, wobei das kolloidale Gold ein Verhältnis der optischen Dichten von 1,7 bis 3,0, gemessen als das Verhältnis der optischen Dichte bei 540 nm zu der optischen Dichte bei 600 nm, aufweist.

**9.** Verfahren nach Anspruch 8, wobei das Verhältnis 2 bis 2,5 ausmacht.

**10.** Verfahren nach einem der Ansprüche 1 bis 9, wobei der Membranstreifen aktiviertes Nylon 66, aktiviertes aufsaugendes Papier oder aktiviertes Polyvinylidendifluorid enthält.

**11.** Verfahren nach einem der Ansprüche 1 bis 10, wobei das eine Ende des Membranstreifens mit einer Waschlösung in Berührung gebracht wird, nachdem es mit der Probe in Berührung gebracht worden ist.

**12.** Verfahren nach einem der Ansprüche 1 bis 11, welches weiterhin die Stufe des Inkubierens dieser Membran bei Zimmertemperatur während einer Zeitspanne von weniger als etwa 10 min vor der Stufe des Beobachtens umfaßt.

**13.** Verfahren nach einem der Ansprüche 1 bis 12, wobei das gesamte Verfahren in weniger als etwa 30 s durchgeführt wird.

**14.** Verfahren nach einem der Ansprüche 1 bis 13, wobei dieser Membranstreifen weiterhin eine Kalibrierung für den nachzuweisenden Liganden unfaßt.

**15.** Verfahren nach einem der Ansprüche 1 bis 14, wobei der Membranstreifen eine Mehrzahl von verschiedenen Liganden-Bindungspartnern aufweist, welche innerhalb der Membran in getrennten Zonen immobilisiert sind, wodurch eine Mehrzahl von Liganden nachgewiesen werden kann.

**16.** Kit für den Gebrauch in einem Test für den Nachweis eines Liganden in einer flüssigen Probe, nach Anspruch 1, welcher Kit unfaßt: eine Membran, welche einen Liganden-Bindungspartner oder einen Liganden zum Binden an den Liganden oder an den Liganden-Bindungspartner aufweist, welcher Liganden-Bindungspartner oder Ligand kontinuierlich, durch die gesamte Membran hindurch, oder in getrennten Zonen immobilisiert ist; und einen Behälter, der einen mit kolloidalem Gold markierten Liganden oder Liganden-Bindungspartner zum Binden an den Liganden-Bindungspartnern auf der Membran in einem Wettbewerbstest, oder zum Binden an den Liganden in der flüssigen Probe in einem Sandwich-Test, enthält.

**Revendications**

**1.** Procédé pour détecter un ligand dans un échantillon comprenant les étapes consistant à :

a) se pourvoir d'une bande de membrane traversée d'un trajet tortueux, ladite membrane ayant deux extrémités avec un ligand, ou un partenaire se liant à un ligand, immobilisé dans la membrane :

b) se pourvoir d'un réactif liquide contenant un partenaire se liant à un ligand, ou un ligand, ledit partenaire se liant à un ligand, ou ledit ligand, étant marqué directement ou indirectement avec de l'or colloïdal ;

c) mettre en contact une extrémité de ladite bande de membrane avec ledit échantillon et, simultanément ou successivement, avec ledit réactif liquide, de façon à réaliser une réaction immunologique lorsque l'échantillon et le réactif se déplacent de ladite extrémité vers l'autre extrémité de la bande de membrane ; et

d) observer ladite membrane pour rechercher la présence d'une couleur dont la distance à partir de ladite extrémité peut être rattachée à la présence d'or colloïdal et dudit ligand dans ledit échantillon.

**2.** Procédé selon la revendication 1, dans lequel :

dans l'étape b), on utilise un ligand, marqué directement ou indirectement avec de l'or colloïdal, qui entre en compétition avec le ligand dans l'échantillon relativement au partenaire se liant à un ligand ; et le réactif et l'échantillon sont mélangés avant leur application simultanée à ladite extrémité de la bande de membrane.

**3.** Procédé selon la revendication 1, dans lequel le ligand est un antigène ayant un site épitopique unique

;
le ligand dans l'étape a) est un antigène ayant la même réactivité immunologique que ledit antigène de l'échantillon ;
le partenaire se liant à un ligand dans l'étape b) est un anticorps marqué directement ou indirectement avec de l'or colloïdal qui est spécifique de l'antigène de l'échantillon ; et
le réactif et l'échantillon sont mélangés avant leur application simultanée à ladite extrémité de la bande de membrane.

4. Procédé selon la revendication 1, dans lequel :
le ligand dans l'échantillon est un anticorps ;
le partenaire se liant à un ligand dans l'étape a) est un antigène pour lequel l'anticorps est spécifique ; et
le partenaire se liant à un ligand dans le réactif est une immunoglobuline, marquée directement ou indirectement avec de l'or colloïdal, qui est spécifique dudit anticorps.

5. Procédé selon la revendication 1, dans lequel :
le ligand dans l'échantillon est un antigène ayant au moins deux sites épitopiques ;
le partenaire se liant à un ligand dans l'étape a) est un anticorps qui est spécifique d'un premier site épitopique sur l'antigène ; et
le partenaire se liant à un ligand dans l'étape b) est un anticorps, marqué directement ou indirectement avec de l'or colloïdal, qui est spécifique d'un second site épitopique sur l'antigène.

6. Procédé selon la revendication 1, dans lequel :
le ligand dans l'échantillon est un antigène ayant au moins deux sites épitopiques ;
le ligand dans l'étape a) est un antigène ayant la même réactivité immunologique que ledit antigène de l'échantillon ;
le partenaire se liant à un ligand dans l'étape b) est un anticorps, marqué directement ou indirectement avec de l'or colloïdal, qui est spécifique des antigènes ; et
le réactif et l'échantillon sont mélangés avant leur application simultanée à ladite extrémité de la bande de membrane.

7. Procédé selon l'une quelconque des revendications 1 à 3, 5 et 6, dans lequel l'antigène de l'échantillon est l'hormone lutéinisante (LH).

8. Procédé selon l'une quelconque des revendications 1 à 7, dans lequel ledit or colloïdal a un rapport de la densité optique de 1,7 à 3,0, mesuré comme le rapport de la densité optique à 540 nm à la densité optique à 600 nm.

9. Procédé selon la revendication 8, dans lequel le rapport est de 2 à 2,5.

10. Procédé selon l'une quelconque des revendications 1 à 9, dans lequel la bande de membrane comprend du Nylon 66 activé, du papier absorbant activé ou du poly (difluorure de vinylidène) activé.

11. Procédé selon l'une quelconque des revendications 1 à 10, dans lequel ladite extrémité de la bande de membrane est mise en contact avec une solution de lavage après avoir été mise en contact avec l'échantillon.

12. Procédé selon l'une quelconque des revendications 1 à 11 comprenant de plus l'étape d'incubation de ladite membrane à la température ordinaire pendant une période inférieure à environ 10 minutes avant ladite étape d'observation.

13. Procédé selon l'une quelconque des revendications 1 à 12, dans lequel la totalité du procédé est réalisée en moins d'environ 30 secondes.

14. Procédé selon l'une quelconque des revendications 1 à 13, dans lequel ladite bande de membrane comprend de plus un dispositif d'étalonnage pour ledit ligand à détecter.

15. Procédé selon l'une quelconque des revendications 1 à 14, dans lequel ladite bande de membrane a plusieurs partenaires se liant à un ligand différents immobilisés dans la membrane en des zones séparées pour que plusieurs ligands puissent être détectés.

16. Nécessaire pour l'emploi dans un essai pour la détection d'un ligand dans un échantillon liquide selon la revendicatin 1 comprenant : une membrane dans laquelle a été immobilisé, en continu ou dans des zones séparées, un partenaire se liant à un ligand, ou un ligand, pour la liaison respectivement au ligand, ou au partenaire se liant à un ligand ; et un récipient contenant un ligand, ou un partenaire se liant à un ligand, marqué à l'or colloïdal pour la liaison au partenaire se liant à un ligand sur la membrane dans un essai compétitif, ou au ligand dans l'échantillon liquide dans un essai en sandwich.